# EUROPEAN PATENT SPECIFICATION

(11) **EP 3 685 402 B1**
(45) Date of publication and mention of the grant of the patent: **26.06.2024**
(21) Application number: 18804719.5
(22) Date of filing: 19.09.2018
(51) Int. Cl.: G16H 50/20, G16H 50/30

(54) **CORRELATION METHOD BETWEEN ALLERGIC SYMPTOMS AND ALLERGENS**
KORRELATIONSVERFAHREN ZWISCHEN ALLERGISCHEN SYMPTOMEN UND ALLERGENEN
PROCÉDÉ DE CORRÉLATION DE SYMPTÔMES ALLERGIQUES ET D'ALLERGÈNES

(30) Priority: 23.09.2017 IT 201700106570; 23.09.2017 IT 201700106602
(43) Date of publication of application: 29.07.2020
(73) Proprietor: TPS Production Srl, 00141 Roma (RM) (IT)
(72) Inventor: TRIPODI, Salvatore, I-00141 Roma (RM) (IT); PELOSI, Simone, I-00165 Roma (RM) (IT); COSTA, Corrado, I-00199 Roma (RM) (IT); BRIGHETTI, Maria Antonia, I-00028 Subiaco (RM) (IT); TRAVAGLINI, Alessandro, I-00136 (IT)
(74) Representative: Uexküll & Stolberg
(86) International application number: PCT/IT2018/000119
(87) International publication number: WO 2019/058401

(56) References cited:
- EP-A1- 2 388 723
- WO-A1-2009/070122
- US-A1- 2016 026 765
- SHINJI OTANI ET AL: "Associations Between Subjective Symptoms and Serum Immunoglobulin E Levels During Asian Dust Events", INTERNATIONAL JOURNAL OF ENVIRONMENTAL RESEARCH AND PUBLIC HEALTH, vol. 11, no. 8, 29 July 2014 (2014-07-29), pages 7636-7641, XP055487041, DOI: 10.3390/ijerph110807636
- MAN-LI CHANG ET AL: "Analysis of total immunoglobulin E and specific immunoglobulin E of 3,721 patients with allergic disease", BIOMEDICAL REPORTS MAY 2014 SPANDIDOS PUBLICATIONS GBR, vol. 3, no. 4, 29 April 2015 (2015-04-29), pages 573-577, XP055486698, Greece ISSN: 2049-9434, DOI: 10.3892/br.2015.455
- DANIEL CHUNG ET AL: "The significance of serum total immunoglobulin E for in vitro diagnosis of allergic rhinitis : The significance of serum total IgE", INTERNATIONAL FORUM OF ALLERGY & RHINOLOGY, vol. 4, no. 1, 12 November 2013 (2013-11-12), pages 56-60, XP055486415, Oxford ISSN: 2042-6976, DOI: 10.1002/alr.21240
- JOANA VITTE ET AL: "Multivariate Analysis As a Support for Diagnostic Flowcharts in Allergic Bronchopulmonary Aspergillosis: A Proof-of-Concept Study", FRONTIERS IN IMMUNOLOGY, vol. 8, 22 August 2017 (2017-08-22), XP055487046, CH ISSN: 1664-3224, DOI: 10.3389/fimmu.2017.01019
- Jakob Florack ET AL: "Comparison of six disease severity scores for allergic rhinitis against pollen counts a prospective analysis at population and individual level", Pediatric Allergy and Immunology, vol. 27, no. 4, 1 June 2016 (2016-06-01), pages 382-390, XP055486752, GB ISSN: 0905-6157, DOI: 10.1111/pai.12562
- Shinji Otani ET AL: "Associations Between Subjective Symptoms and Serum Immunoglobulin E Levels During Asian Dust Events", International Journal of Environmental Research and Public Health, vol. 11, no. 8, 29 July 2014 (2014-07-29), pages 7636-7641, XP055487041, DOI: 10.3390/ijerph110807636
- Daniel Chung ET AL: "The significance of serum total immunoglobulin E for in vitro diagnosis of allergic rhinitis : The significance of serum total IgE", International forum of allergy & rhinology, vol. 4, no. 1, 12 November 2013 (2013-11-12), pages 56-60, XP055486415, Oxford ISSN: 2042-6976, DOI: 10.1002/alr.21240
- Man-Li Chang ET AL: "Analysis of total immunoglobulin E and specific immunoglobulin E of 3,721 patients with allergic disease", BIOMEDICAL REPORTS MAY 2014 SPANDIDOS PUBLICATIONS GBR, vol. 3, no. 4, 29 April 2015 (2015-04-29), pages 573-577, XP055486698, Greece ISSN: 2049-9434, DOI: 10.3892/br.2015.455
- Joana Vitte ET AL: "Multivariate Analysis As a Support for Diagnostic Flowcharts in Allergic Bronchopulmonary Aspergillosis: A Proof-of-Concept Study", Frontiers in Immunology, vol. 8, 22 August 2017 (2017-08-22), XP055487046, Lausanne, CH ISSN: 1664-3224, DOI: 10.3389/fimmu.2017.01019

## Description

### Field of the invention

This invention refers to the area of allergic diseases, most notably the invention refers to new techniques of diagnostics and treatment of allergies, relying on the evaluation of symptoms in a patient, as these are related to hypersensitivity to a particular allergen.

### Background of the invention

Approximately 20% of the population of industrialized countries presents a severe hypersensitivity (allergy) to exposure to antigens present in the surrounding environment. These antigens induce types of immediate and/or delayed reactivity.

In Italy, asthma and allergies affect more than 25% of children and together they are the first cause of chronic childhood pathology. The impact on the quality of life of patients and their families is remarkable. In general, the weight on public health, with the consequent negative repercussions on the health economy, is also enormous. In particular, adherence to the guidelines for assistance is low; the same it is for the use of fundamental therapies, such as specific immunotherapy (the so-called vaccine), otherwise defined as AIT (Allergy Immuno Therapy), which is currently the only intervention that can change the course of the disease, and the main cause of this is the inevitable gap between the demand and supply of specialized assistance in the territory. This gap implies that only a small percentage of patients can be the subject of effective prevention, diagnosis, monitoring and therapy.

Based on these considerations, it is clear that the use of m-Health, i.e. the use of medical-specific apps downloadable on smartphones more and more popular, could represent an effective solution for better patient care with allergic rhinitis, but also for other allergic diseases.

The WO 2009/099379 is a technique for obtaining clinical diagnoses based on the *results* of tests performed with a plurality of biomarkers (in practice the dosage of specific IgE for allergenic molecules) tested. The method is based on data-selection modules and includes the phases related to the acquisition of the results of a series of tests; access to a predefined structure that contains biomarkers associated with hosts (suspected allergic patient)-at least one host is associated with a plurality of biomarkers-; the identification of a group of hosts by mapping the biomarkers tested; the assignment of an evaluation to each host in the group based on the results of the biomarkers tests being evaluated, mapped with reference to the host. Hence this host rating is provided with a second resolution of the values. The indicative data of the host group and the assigned host evaluations are provided as input into a decision database installed on a computer in order to generate a support diagnostic for the clinical decisions. Host structures are envisaged at a differentiated hierarchical level, the number of hosts is expected to be less than the number of biomarkers available and at the same time the second level of resolution is lower than the first level of Resolution. Scaling of test results of host evaluations is based on specific data related to the patient's history. These data include demographic data, medical history, hereditary factors, responses to particular stresses. In practice, the patent WO 2009/099379 refers to the recent use of a set of tests with a plurality of biomarkers used for a single patient. The protocol of execution of the diagnosis involves different modalities, that is to say according to the symptoms, according to the patient's history or according to the diagnosis by the tests. It takes into account that in more recent years the availability of molecular biomarkers has increased exponentially and that more and more work is done with modules that combine the information on the patient's history with the information deriving from the tests, to reach a complex and complete diagnosis. However, when one has to work with hundreds of tests, at the same time, there could be a risk of misinterpretation on the part of the doctor. It was therefore obvious and immediate a solution-as in WO 2009/099379-which tended to bring all these results on a computer application for its processing. In any case, it remains to be selected which type of exams to detect and acquire, which to point out and which to keep in the alternative. This problem is the object of study and research according to this invention. In particular in previous solutions there were no integrations on the curves of symptoms recorded by patients and the curves of pollens and / or other allergens related to the area where the patient resides. As well as they did not provide for integrations on the data relating to the environmental pollutants (PM10, PM5 etc.) that can be detected by ARPA control units and also on the weather variables.

The purpose of this invention is to provide an additional diagnostic approach to refine the diagnosis, and this will be possible by dosing in the blood of patients the antibodies responsible for the allergic reaction (specific IgE) directed to both extracts (the whole of the various molecules that constitute a given allergen) both to specific molecules present in the inhalant allergens, but not only, since the same methodology is also applicable to food allergies, drugs and the poison of Hymenoptera, but integrating these data with different SMS (Symptoms medication Score, i.e. curves of both symptoms and drugs recorded by patients).

A further aim, in fact, concerns the integration with automatic data import systems (clinical history, skin allergometric tests, Component Resolved Diagnosis, i.e. molecular IgE dosage, SMS curves, inhalant allergen count) or user-friendly manual input, all the above elements being combined together by one or more dedicated algorithms to lead to the result of the definition of the fundamental allergens for that patient because they are correlated by a cause-effect relationship to its symptomatology.

Yet one purpose of this invention is to provide multivariate diagnostic approaches to refine the diagnosis, and this will be possible by dosing in the blood of patients the antibodies responsible for the allergic reaction (IgE specific) direct both to all allergenic extracts, in toto, and to specific molecules present in pollens and/or other allergens, also present in foods, drugs, etc.

A further purpose concerns the integration with automatic data import systems (clinical history, skin allergy tests, CRD, symptom curves, pollen count, etc.) or user-friendly manual input, all of the above elements being combined together by a dedicated algorithm in order to reach the result of the definition of the basic allergens for that patient.

Finally, the aim of this invention is to provide techniques and devices for multivariate approaches for the study of the relationship between symptoms and allergens, which employ acquisition and analysis protocols and means of research and processing of the recorded data, among the most widespread and recognized as standard in the field of allergic diseases, in order to make the embodiment of the invention immediate, reliable and easy to be employed.

The above-mentioned objectives are achieved by a method according to claim 1. Preferred embodiments are set out in the appending claims 2 to 7.

In order to better clarify the invention and without limiting the scope and the areas in which it can be used, the following will describe some particular embodiments, with reference also to the enclosed drawings.

### Brief description of the drawings

Figure 1a is a pyramid-shaped graph, each relative face corresponding to an allergen, and each relevant sector corresponding to a diagnostic stage.
Figure 1b is a graph of the symptoms and drug (SMS-Symptoms medication Score) curves recorded by patients on the monitoring application.
Figure 2 is a pyramid-shaped chart where the faces related to the allergens of interest with increasing color intensity from the periphery to the center, wherein the relevant allergens are pointed out.
Figure 3 is a representation within a pyramid divided into 5 areas of diagnostic steps, through which there is a gradual reduction in the number of allergens taken examined, until we find those of interest for a AIT, represented in the apical field of the pyramid.
Figure 4 is the graph of the pyramid obtained after the first diagnostic step in the clinical case considered, the evaluation of the local pollen calendar with the coloration of the outermost areas of the faces relative to the possible allergens.
Figure 5 is the graph of the pyramid obtained after the second diagnostic step in the clinical case considered, the skin tests, with the coloring of the sector immediately innermost of the faces relative to the possible allergens.
Figure 6 is the graph of the pyramid obtained after the third diagnostic step in the clinical case considered, dosage of IgE for allergenic molecules, with the coloration of the third sector of the faces relative to possible allergens.
Figure 7a is the graph of pollen count provided by the Tor Vergata aerobiology Center for Graminaceae for the period March-August 2016, recorded in Rome, expressed according to the criteria of PP EAACI (Pfaar) or according to local criteria (Italy).
Figure 7b is the graph for pollen peaks only.
Figure 8 is the graph of the symptoms and medication curves (SMS-Symptoms medication Score) recorded in the monitoring application, relative to the clinical case considered.
Figure 9 is the final pyramid-shaped graph, with faces coloring of the allergens of interest in the clinical case considered, and the apical zone, where these are reported.
Figure 10 shows graphs related to the data of a single patient polisensitive to pollens, but not to the spores of alternaria.

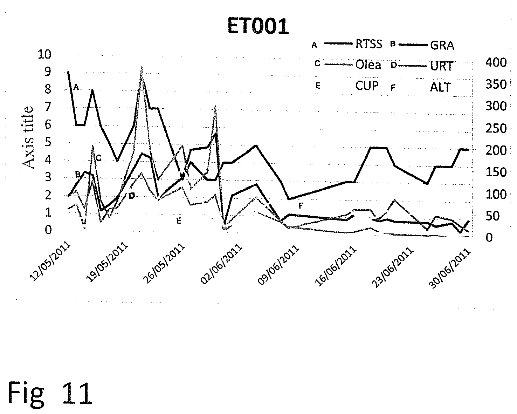

Figure 11 is the graph of the symptom score that represents the trend during the detection period.

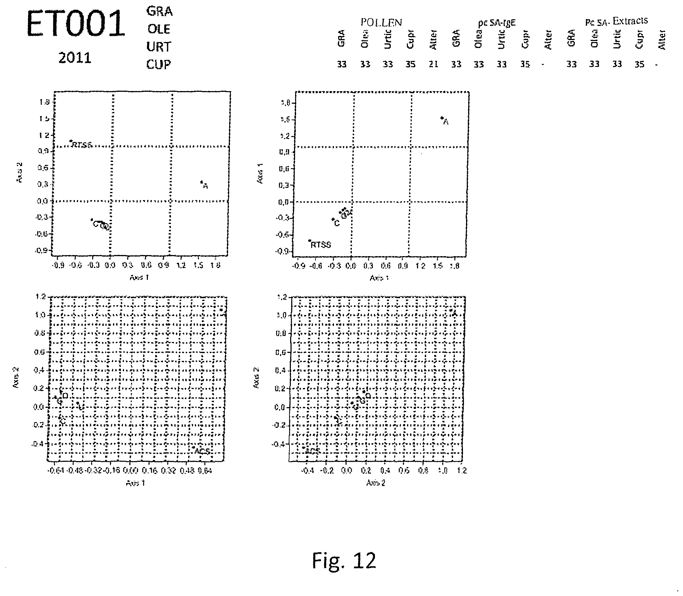

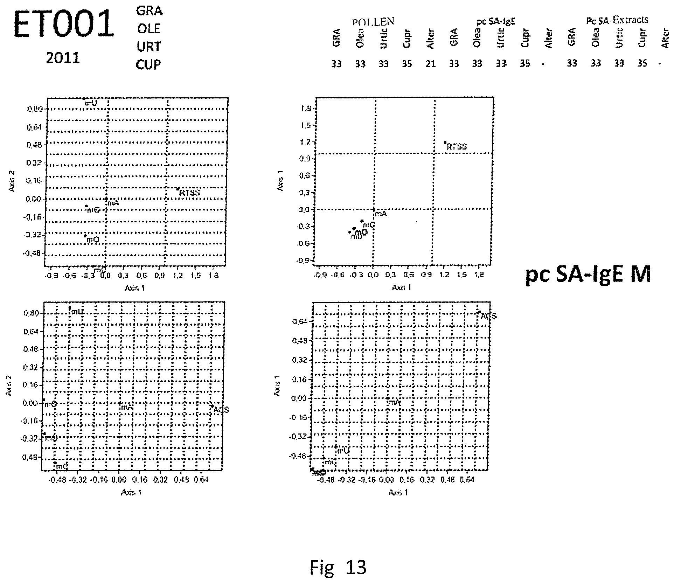

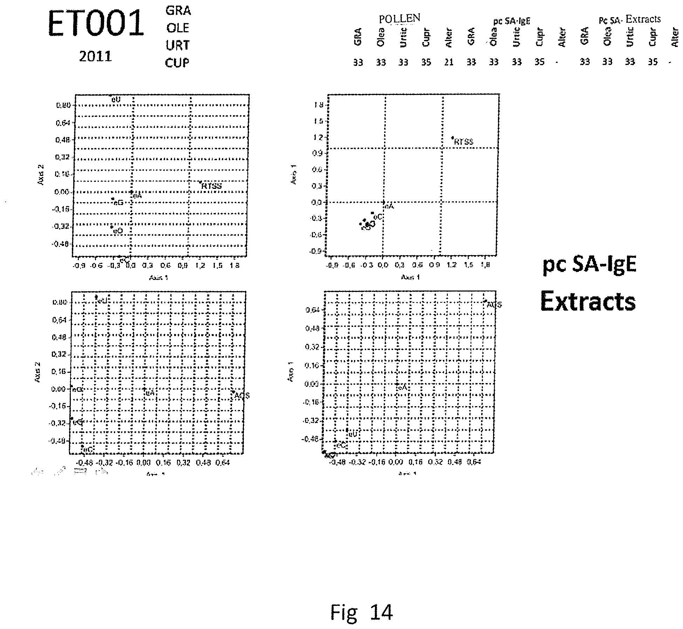

Figures 12, 13 and 14 are representations relative to both the numerical result of the univariate approach and to the graphical result of the multivariate one of the concordance expressed in terms of proximity, between two scores, among the many available, and analyzed in the example reported, RTSS (Rhino Conjunctivitis Total Symptom Score) and ACS (Average Combined Symptom Score), and pollinic variables, considered as absolute variables or correct for Specific Activity (AS, see after) referred to molecular IgE or to extracts.
Figures 15a, 15b, 15c are diagrams related to the examination of a first patient regarding positivity to the tested allergens.
Figures 16a, 16b, 16c are diagrams relating to the examination of a second patient regarding the positivity to the tested allergens.

Further peculiar features of the preferred forms of realization of this invention will be described below for illustrative and non-limiting purposes.

### Detailed description of the invention.

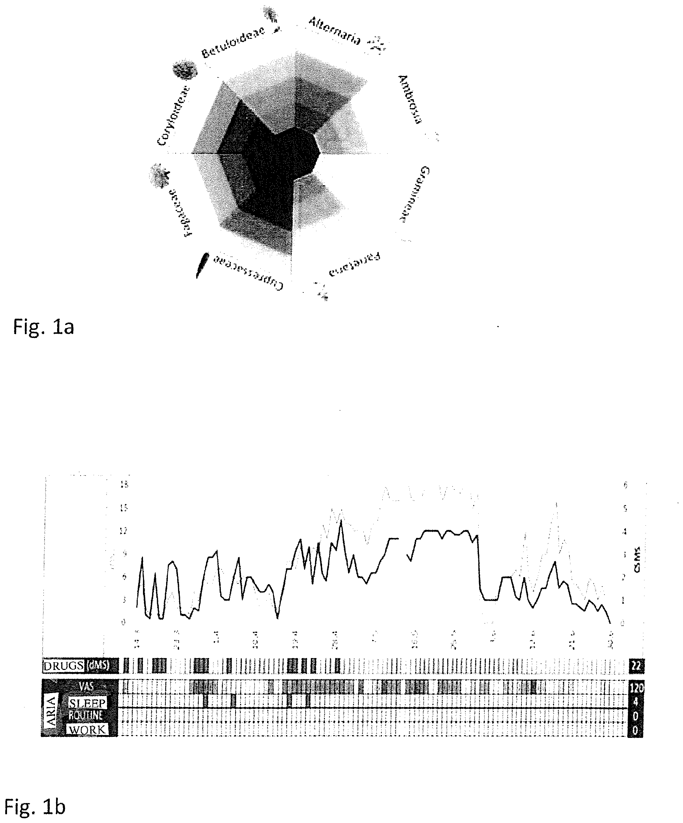

Figure 1a shows the image of the pyramid-shaped chart with multiple sections with central target, obtained in an example clinical case.

A key point for the management of respiratory allergies, and in particular, allergic rhinitis (colds) is the exact identification of inhalant allergens to which the patient is sensitive and, among them, inhalant allergens (pollen, acarus, moulds, epidermal derivatives of animals) dispersed in the air are among the most important, both for the purpose of a possible prevention (avoiding exposure) and to choose the best allergen for the desensitizing vaccine or allergen specific immunotherapy (AIT). This is crucial because AIT is the only therapy that has been proven to be able to change the natural history of allergies. But this is not easily achievable, especially in the Mediterranean area, where pollens are aereodispersed practically in the same period.

Furthermore, today more and more allergic patients are polysensibilized to more allergens. In A recent study (1) on 1360 children from 4 to 18 years of age, 83% was at the same time positive for cutaneous tests (skin prick test, SPT) at least 3 pollens and 50% at 6 pollens. Therefore this requires an additional diagnostic approach to refine the diagnosis, and this is possible by dosing in the blood of patients the antibodies responsible for the allergic reaction (IgE specific) directed towards specific molecules present in the inhalant allergens. In fact, some molecules are present only in that specific inhalant allergen (e.g. Phl p 1 is present only in pollens of gramineae, while Ole and 1 is the genuine molecule of olive pollen. Also in another our publications (2) we have shown that using this method called CRD (Component Resolved Diagnosis) which took into account the presence of specific IgE towards genuine molecules of an inhalant allergen, it would have modified the identification of the exact composition of the vaccine, and therefore the exact sensitization of the patient in more than 50% of the cases, compared to the only clinical history and the results of the SPT.

But this is not enough because even in real polysensitized patients to the genuine molecules of inhalant allergens, sometimes there is no clinical reaction of the patient when breathing them and we could demonstrate this using a dedicated app with which the patient very simply records daily the symptoms in the involved pollinic period and comparing the curves of symptoms (we can use several clinical scores that consider also the taken medicines) with the daily counts of inhalant allergens (3).

The present invention refers to a method allowing to integrate into a procedural application, with automatic data import systems (such as skin allergy test results or laboratory test results of sepcific IgEs) or easy manual input from the user, all of the above elements combined together with one or more dedicated solutions in such a way as to obtain the definition of the basic allergen (s) for that patient.

The report will be produced automatically by the system so that an end result is provided that, with good statistical reliability, provides a useful and real time diagnosis for the patient, easily understandable even by the non-specialist doctor.

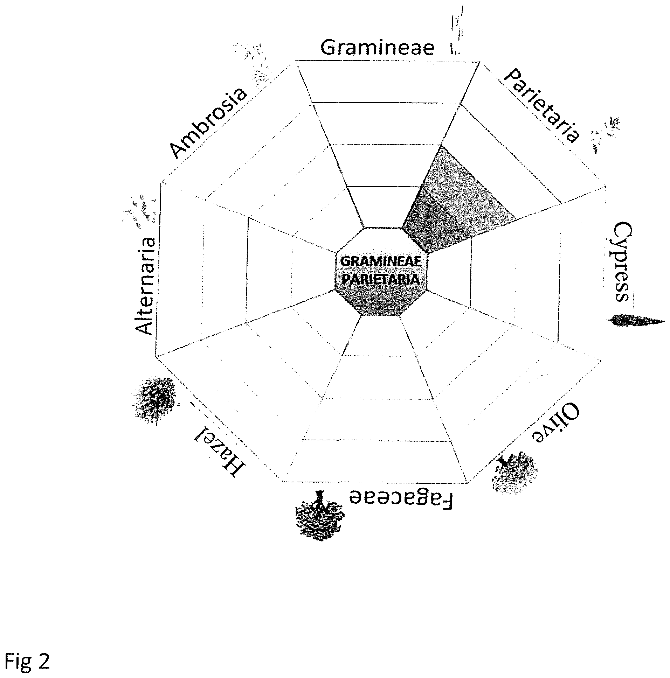

In Figure 2 is represented the result of the diagnostic process through a regular geometric figure that presents as a kind of target in which progressively, also with the intensity of the various colors from the periphery to the center, one gets to "center the target". That is to say that the allergen or allergens relevant to the specific patient will appear in the center, with the integrated assessments of all the data. In the present case there are significant gramineae, in grey, and parietal, in dark grey, reported in the central sector of the target. All other sectors, corresponding to the non-relevant allergens and therefore excluded and coloured in light grey.

Moreover, the allergic skin tests are very widespread for the diagnosis of allergic diseases, derived from inhalant allergens, such as inhalant allergens, derivatives of acarus or domestic animals, moulds, food, both of plant origin and food of animal origin, latex, insect venom, drugs (the diagnosis of these allergies also includes intradermal cutaneous tests). The answer we usually get is that the patient is "sensitized" (such diction does not mean that the patient is allergic). The term "sensitized" indicates that a certain allergizing substance is recognized but it is not sure that the patient has the "clinical" symptomatology. That is to say that the patient may not have any manifestation if he comes into contact with the involved substance. The answer can therefore be very different depending on the extent of the skin response (wheal diameter) to the SPT, a response that can vary for example even from 0 to 20 mm. This is the reason that we choose a certain value (cut-off) to which a given predictive positive meaning can be conferred, knowing that the minimum value is 3 mm. The same applies to laboratory analyses, or data related to the patient's clinical response to exposure to the breathing allergen.

Putting together all these data at various levels of choice of input parameters, with different weights, variables, which can also be selected by the user himself (both patient and medical) or presented on the basis of guidelines, gives the answer that ascertains whether the patient is, or is not, allergic. Therefore the user ( the physician or the patient) by modulating the different cut-offs (i.e. the discriminating value, or threshold value) for the various tests (SPT, molecular IgE dosage, total IgE, SMS intensity, pollen count and/or other inhalant allergens, environmental pollutants, climatic weather data) differently combined, can regulate (tuning) the sensitivity and specificity of the final response: correlation of the SMS with the count of inhalant allergens.

In other words, we have set the object to carry out a large series of examinations and to integrate them, together, in an automatic way, by means of a dedicated application.

In fact the program records the patient data through an application, for the recording of the symptoms (Allergycard Web) and, downstream of the recording, to determine the weight to be assigned to each data acquired, a series of scores are available and a series of validated clinical scores at international level to achieve a truthful diagnosis referred to a time period. All this is set in correlation with the count of daily inhalant allergens, so that the data are integrated with each other. In this context, the skin responses (SPT) and laboratory data are inserted, together with specific IgE dosage for extracts and/or molecules (which can be manually inserted, or the laboratory sends them automatically and the results are recorded in the system). Then based on all the acquired data and based on the preset values that have been entered, we get the final response with the diagnosis.

Without going into detail, it is just this the sequence of steps (a) to (c) according to claim 1 that characterizes the method according to the present invention: by integrating all these data, processed by means of one or more specific algorithms, in the end comes out the diagnosis of the patient, who then-as aforementioned- is not only relative to inhalant allergens but it can also be referred to a food allergy, insect venom, drugs, etc.

If it is detected that more data is concentrated in the same direction, it is established for certain, that the allergy depends on a specific substance because it has been revealed the increased sensitivity to that allergen. Lastly, the colorimetric thickening, as shown in Figure 2, on a particular field of the Chromatographic visualization scheme, allows to highlight the different distribution and the different reaction to the different sensitizations.

The following pages will cover the applicable pattern in the case of allergic Rino-conjunctivitis and/or allergic asthma from inhalant allergens.

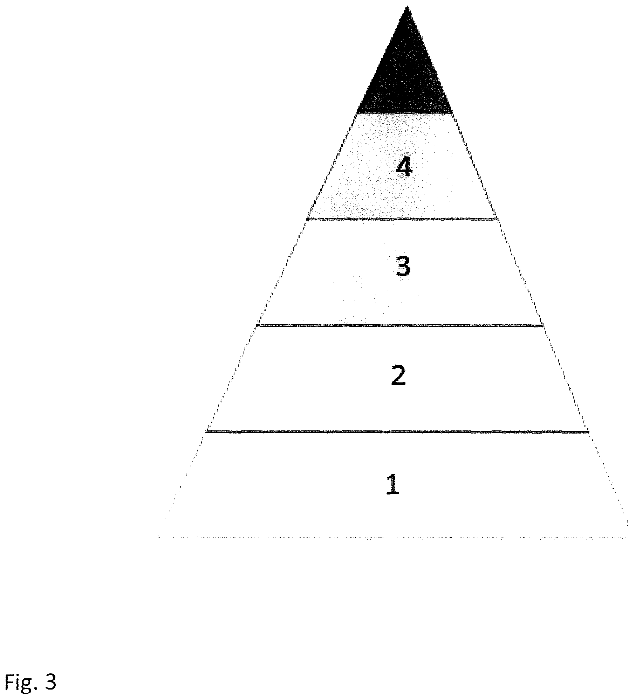

Through a series of operational steps (integrated in an algorithm that processes them automatically) the system analyzes the various data in successive steps, according to the following sequence (also illustrated in Figure 3):
1. Clinical history and local Pollinic calendar
2. Results of skin tests (SPT)
3. Results of IgE for extracts and for genuine allergenic molecules.
4. SMS related with the curves of inhalant allergens (pollens and molds) recorded by the patient based on its clinical history and sensitizations.

Figure 3 illustrates the various diagnostic steps in a patient with suspected pollinosis (often highly polysensitive), arranged in order from the base to the apex of a pyramid divided into 5 coloured sectors with increasing intensity:
1. Seasonality of symptoms. Diagnostic hypothesis.
2. SPT or IgE test with extracts. Atopic sensitization.
3. Molecular IgE test. Sensitization for genuine molecules
4. E-Diary SMS and pollen count. Clinical relevance

The steps are characterised by a gradual reduction in the number of pollen extracts taken into account for AIT, represented in the fifth sector of the pyramid, namely the apical sector.

Through these various phases will be excluded the possible apparent initial sensitizations in order to arrive, in the model that therefore has been defined as pyramidal exclusion, in the center of the pyramid where the clinically relevant allergens will be reported for that patient.

To clarify the functionality of the system there are some clinical cases, to which the method has been applied.

### Clinical Case 1

### Step 1: Clinical History and local Pollinic calendar

### Case 1, M, 13 AA

| Allergic rhinitis | Terapia | Others |
|---|---|---|
| Persistent | Anti-H1 topic | Allergic Familiarity |
| Moderate-Severe | Anti-H1 oral | Atopia per allergeni perenni |
| Blocker | Topic steroid | No animals at home |
| Onset age: 7 years | Oral Steroid | |
| Conjunctivitis: no | | |

Child with a picture of obstructive rhinitis (Blocker) based on symptoms of moderate - severe persistent degree, according to internationally known criteria of guidelines ARIA.

Based on the local pollen calendar, where H (high) is highlighted with periods of greater allergenic concentration, M (middle) highlights periods of medium allergen concentration and L (low) are highlighted periods of low allergengenic concentration, the symptomatology that is reported, highlighted in grey, occurs in the months that go from March to September.

| | | | | | | | | | | | | |
|---|---|---|---|---|---|---|---|---|---|---|---|---|
| ROME | G | F | M | A | M | G | L | A | S | O | N | D |
| CYPRESS | L | H | H | L | L | | | | | L | L | L |
| PARIETARIA | L | L | M | H | H | M | M | L | L | L | L | L |
| HAZEL | L | L | M | M | L | | | | | | | L |
| FAGACEAE | | | | H | H | M | L | | | | | |
| GRAMINEAE | | | L | H | H | H | M | L | L | L | L | |
| OLIVE | | | | | H | H | | | | | | |
| ALTERNARIA | | | | | L | M | M | M | H | L | | |
| AMBROSIA | | | | | | | | | L | | | |
| PATIENT | | | | | | | | | | | | |

Therefore, in accordance with this first step, the following inhalant allergens should be considered: Cypress, Parietal, Beech, Gramineae, Olive and Alternaria.

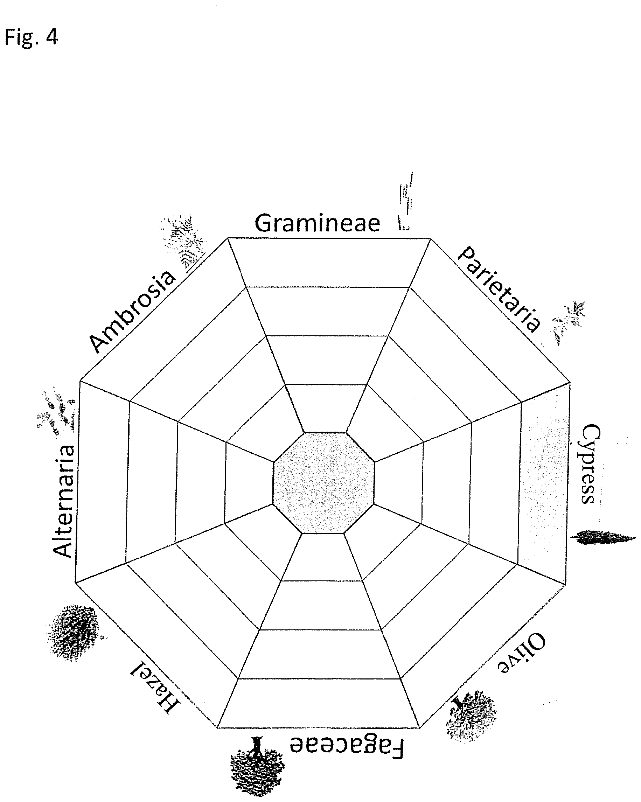

In Figure 4 is represented the pyramid of the model in which the outermost band was coloured, relative to all possible inhalant allergens, with the exception of Ambrosia, whose sector is in fact highlighted in grey, precisely because pollen is not present in the specific area.

### Step 2: Results of skin tests (SPT)

The patient was administered SPT for suspected allergens and the result is expressed in mm relative to the diameter of the skin response (wheal), taking into account that 3 mm is the minimum value considered positive. The user, as stated above, can choose the value of the cut-off in mm that he wishes to choose, in his opinion, what allows him to have the best ratio of sensitivity/specificity. The algorithm, depending on the value chosen will give different results and the same happens for the next steps based on the values of the specific IgE and the intensity of the SMS. This is the so-called "tuning" of the system by the user. The result of cutaneous allergic tests in the case of patient No. 1 is reported below.

| SPT | ALLERGEN |
|---|---|
| 6,0 | PARIETARIA |
| | Par J 2 |
| 10,5 | GRAM (PHL) |
| | Phl p 1 |
| 8,5 | GRAM (CYN) |
| | Cyn d 1 |
| 4,5 | AMBROSIA |
| | Amb a 1 |
| 0,0 | ALTERNARIA |
| | Alt a 1 |
| 3,0 | HAZEL |
| | Cor a 1 |
| 4,0 | FAGACEAE |
| | Bet v 1 |
| 4,0 | OLIVE |
| | Ole 1 |
| 3,0 | CYPRESS |
| | Cup a 1 |
| PROFILINE | Phl p 12 |
| | Bet v 2 |
| POLCALCINE | Phl p 7 |
| | Bet v 4 |
| CARBOHYDRATES | CCD |

| | |
|---|---|
| 0,0 | ACARUS |
| 6,0 | CAT |
| 3,5 | DOG |

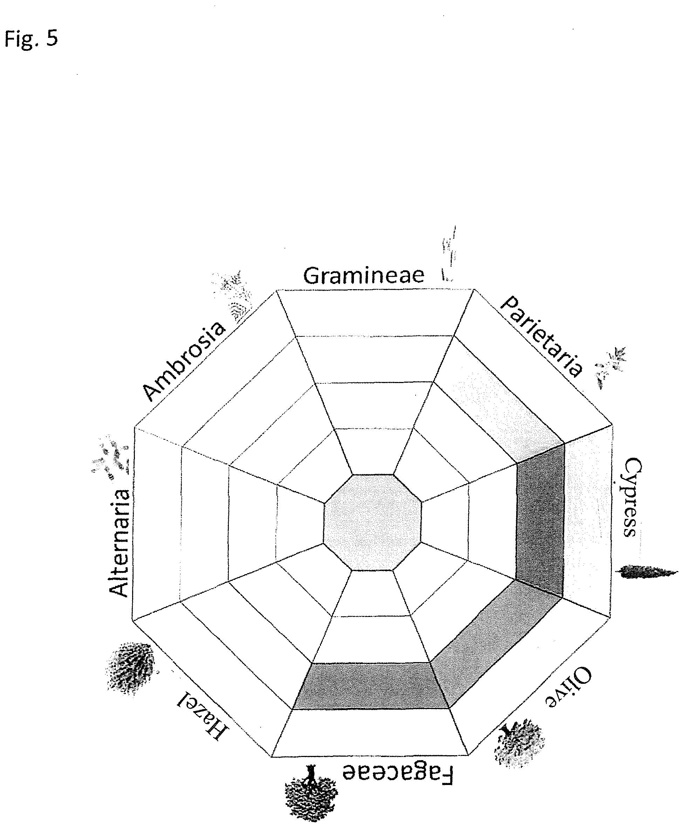

In Figure 5 the change of the decision pyramid after the second step is shown, choosing a threshold value of 3 mm. It was coloured, with a higher colour hue than the outermost band, the second sector of the sections related to the allergens of interest. Having detected a negative SPT for the Alteraria, the relevant sector is coloured in grey to indicate its exclusion among the possible inhalant allergens selected (pyramidal exclusion algorithm).

### Step 3: IgE results for allergen molecules.

This step examines the laboratory's response (on blood sampling, but not only, since other biological fluids can be dosed, such as nasal secretes, tears, etc.) with the dosage of specific IgE for the genuine molecules of an inhalant both of those cross-reactive (panallergen) allowing to identify true sensitizations from false ones. Also in this case the result will be different depending on the chosen cut-off.

The analysis of the molecular IgE shows a true sensitization only for the Gramineae (Phl p1 positive) and for the parietary (Par J 2 positive), it also highlights the presence of IgE for the panallergene Profiline (Phl p 12 and Bet v 2 positive) explaining the false Positivity of SPT.

| SPT | ALLERGEN | slgE |
|---|---|---|
| 6,0 | PARIETARIA | 18,0 |
| | Par J 2 | 35,0 |
| 10,5 | GRAM (PHL) | 90,0 |
| | Phl p 1 | 80,0 |
| 8,5 | GRAM (CYN) | 2,4 |
| | Cyn d 1 | 0,4 |
| 4,5 | AMBROSIA | 0,1 |
| | Amb a 1 | 0,1 |
| 0,0 | ALTERNARIA | 0,1 |
| | Alt a 1 | 0,0 |
| 3,0 | HAZEL | |
| | Cor a 1 | 0,1 |
| 4,0 | FAGACEAE | 0,1 |
| | Bet v 1 | 0,1 |
| 4,0 | OLIVE | 0,4 |
| | Ole 1 | 0,1 |
| 3,0 | CYPRESS | 0,1 |
| | Cup a 1 | 0,1 |
| PROFILINA | Phl p 12 | 17,4 |
| | Bet v 2 | 29,0 |
| POLCALCINA | Phl p 7 | 0,1 |
| | Bet v 4 | 0,1 |
| CARBOHYDRATES | CCD | 1,0 |

| | |
|---|---|
| 0,0 | ACARUS |
| 6,0 | CAT |
| 3,5 | DOG |

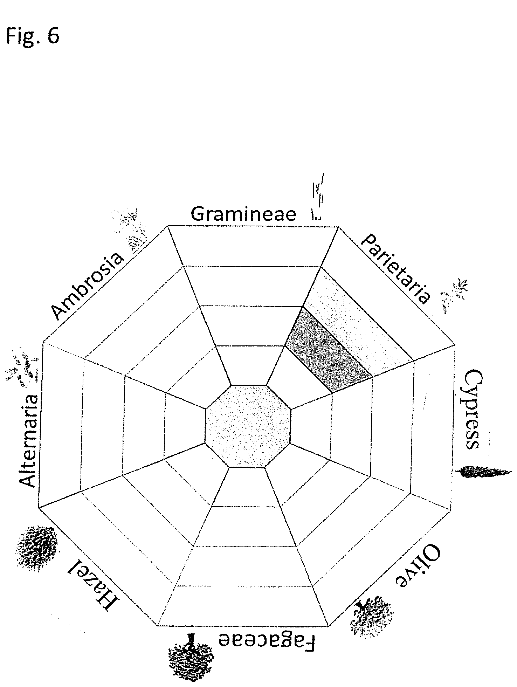

In Figure 6 is represented the decision pyramid , in which, being positive the IgE only for gramineae and parietaria, the third sector of the relevant sections is coloured with even more intense tones, while the remaining sections are colored in light grey to indicate the exclusion of relative allergens.

Therefore, the methodology of pyramidal exclusion, allows to restrict even more the number of potential allergens, approaching the center of the target.

Step 4: Utilizing of the patient symptom recording data through the dedicated monitoring app, integrated with pollen curves. Below it will be schematically described the functionality of the monitoring application, but, as a matter of fact, the entire system of the CDSS (Clinical Decision Support System) can be integrated into a single app.

### Description of the monitoring application

The patient's electronic clinical diary is carried out through the smartphone monitoring application. The application allows the recording of symptoms of allergic rhinoconjunctivitis and asthma, the intake of antiallergic or anti-asthmatic drugs and the adhesion to the sublingual immunotherapy (SLIT). The patient's access to the monitoring application is executed through the personal identification and a keyword provided by the attending physician and/or the laboratory where the molecular IgE is taken; the patients are explained how to download (from the Google Play stores, for Android systems, or from the Apple store, for IOS Systems) and install the app on his Smart-Phone, and they receive briefly the instructions on how it works. While registering, patients provide consent to the processing of data, in compliance with the current privacy policy and security of sensitive data. Patients are required to have daily access to the application during the assigned monitoring period, in order to accurately record the severity of the ocular, nasal and respiratory symptoms on a zero-to-three scale through the use of emoticons, answer questions about quality of life (QoL), record the intake of symptomatic drugs and express daily through a VAS (visual analogue scale) the state of wellbeing.

The insertion of daily data takes a few minutes and is easy and intuitive for children as emoticons and Visual Analog Scale (VAS) are usually employed.

Doctors can evaluate their patient data on a web backoffice, after personilezed login. On this back-office the data are recorded both in the form of raw data and as graphs representing different clinical scores, internationally validated:
- RTSS rhinoconjunctivitis Total Symptom Score
- ACS Average Combined Score
- AdSS Average Adjusted Symptom Score
- RMS Rescue Medication Score
- dCSMS Daily Combined Symptom and Medication Score
- Asthma Score

These scores of clinical severity, graphically represented, can be compared with the pollen curves, thus making it evident a possible reciprocal correlation.

Moreover, through the daily data collection, the monitoring application allows the exact classification of the allergic rhinitis of the patient according to the ARIA criteria, exceeding the limits of a medical history recorded retrospectively.

During the monitoring period the nearest aerobiological centre where the patient resides, provides pollen count expressed in granules/m³ of air, sending them automatically to the Management Server on a weekly basis. The daily concentrations of pollen granules are usually sampled with a VPPs ^{®} 2000 (Volumetric Pollen and Particle Sampler, Lanzoni S.R.L., Bologna) instrument placed at about 15 m height referred to the road plan.

### Pollinic Curves

On the basis of data about pollen and spore concentrations provided by local aerobiology services, periods of pollination for each species are established from year to year. The method for selecting these time periods is not standardized because of the many variables involved and the only reference document is represented by an EAACI Position Paper of 2017 (4). Depending on the circumstances, it may be considered: a) the entire season (pollen season); (b) peak period (peak pollen period); c) days with high concentrations (high pollen days). The cut-offs used to establish these periods, as far as possible, refer to those indicated in the Position Paper, with the possibility of adapting to the local pollinic situation.

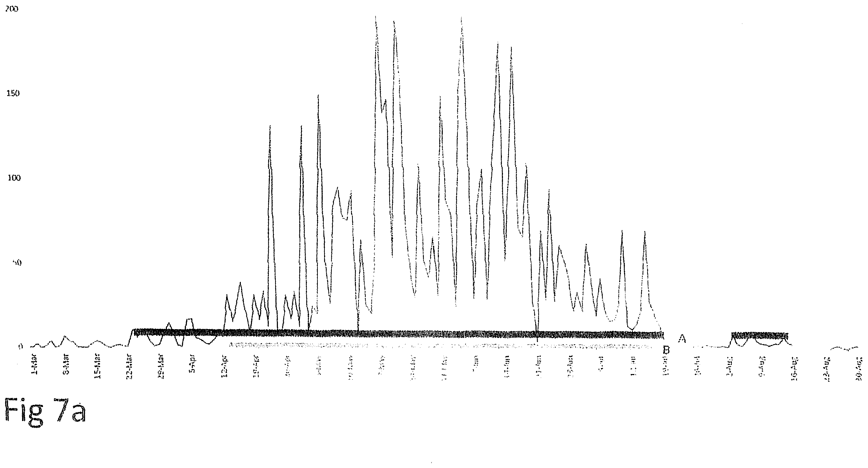

The following table refers to Figure 7a, which shows the graph of the pollen count provided by the Tor Vergata Center of Aerobiology for the Gramineae for the period March-August 2016 in Rome, and they are expressed or according to the criteria of PP EAACI (Pfaar) or According to local criteria (Italy) more restrictive and more adapted to the Italian reality.

| **SEASON** | **Criteria** | **duration** | **Start 1** | **End 1** | **Start** 1 | **End 2** | **Adherence** |
|---|---|---|---|---|---|---|---|
| **Pfaar** | 5 days (out of 7 consecutive days) each with ≥3 pollen/m3 and with a sum of ≥30 pollen/m3 | **132 dd** | 23-Mar | 19-Jul | 3-Aug | 15-Aug | 82% |
| **Italy** | 5 days (out of 7 consecutive days) each with ≥10 pollen/m3 and with a sum of ≥100 pollen/m3 | **97 dd** | 13-Apr | 18-Jul | \ | \ | 82% |

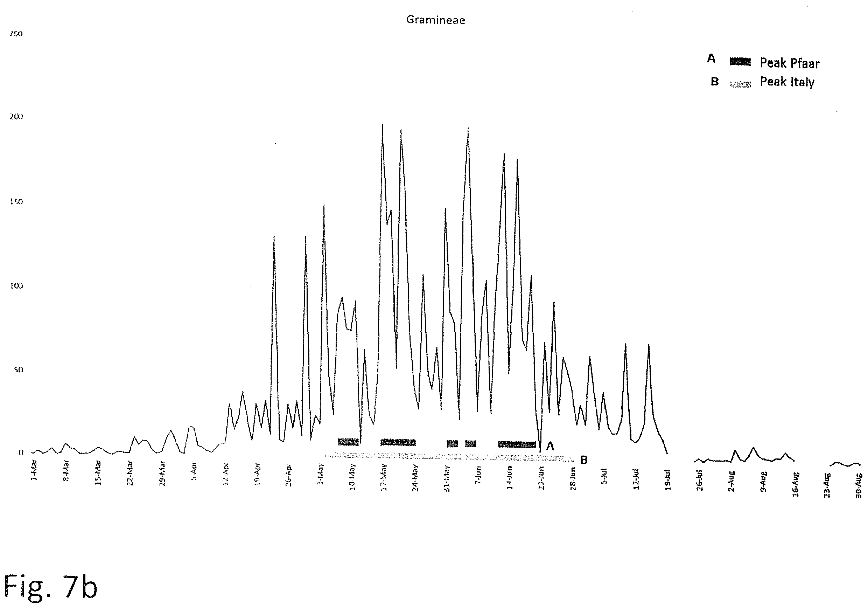

The following table refers to Figure 7b, which shows the data and the graph for pollen peaks only.

| **PEAK** | **Criteria** | **duration** | **Start** | **End** | **Adherence** |
|---|---|---|---|---|---|
| **Pfaar** | 3 consecutive days, each with ≥50 pollen/m3 | **28 dd** | 5 intervals | | 81% |
| **Italy** | 3 days (out of 5 consecutive days) each with ≥50 pollen/m3 | **55 dd** | 4-May | 28-Jun | 81% |

The CDSS system automatically determines which period to analyse on the basis of the previously set ("tuning") criteria (whole season, pollen peak, days with higher pollen concentrations).

Each patient receives from the doctor/laboratory indications on the exact period in which to fill in the electronic diary of symptoms (compilation period). Adherence to the compilation of the electronic diary is calculated by reporting the days actually compiled by the patient to the total of the prescribed ones. This ratio can be considered satisfactory if higher than 67%-70%.

Each patient compiles the electronic diary daily through the monitoring application, answering a set of questions about his symptomatology and the medications he/she has used. To each question is assigned a score that is used to process the following indices (or SMS, Symptom and Medication Score): a) RTSS (Rhinoconjuctivits Total Symptom Score), which affects only the symptoms; b) CSMS (Combined Symptom and Medication Score), which relates to both symptoms and medications. In addition, the impact of rhinoconjunctivitis on the patient's state of health is evaluated by a VAS (Visual Analogue Scale). The cut-offs of these indices to determine if the symptoms are related to exposure, or not, to a particular pollen (or spore), are not standardized. By means of these procedures, each physician may decide to use a different cut-off based on the patient's context and clinical history.

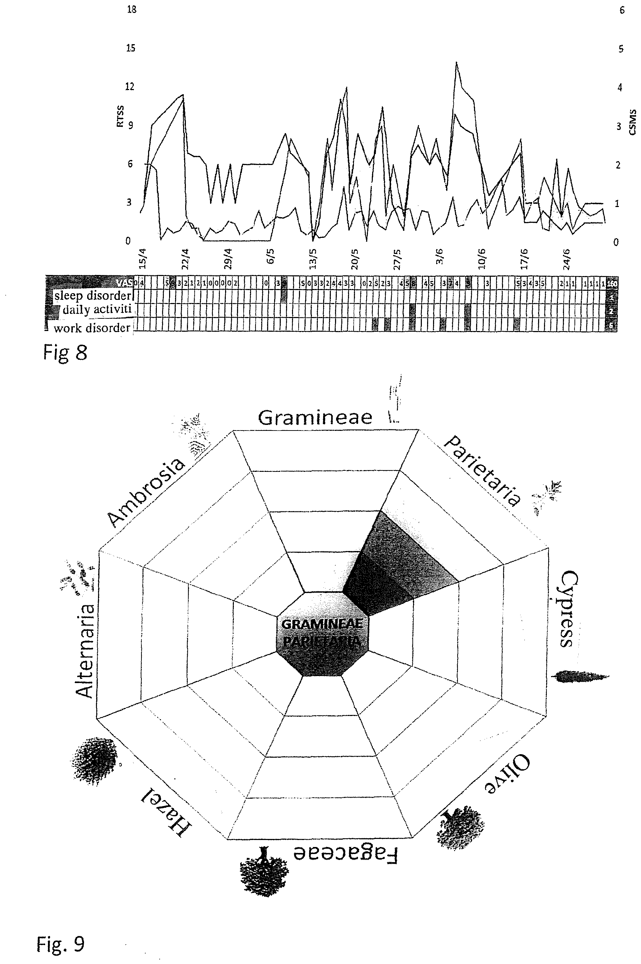

Figure 8 shows the resulting graph of the data analysis of Case No. 1, in a period between 15/04 and 01/07, represented in the abscissa, while in the ordinate are represented on the left the RTSS values and on the right the CSMS values. The table below shows the data regarding VAS, sleep disturbance, daily activities and labor disturbance, for each day of the month and total, with dark blue coloration for the days of the month in which these data are relevant. All the parameters indicated in such a way are analyzed to allow the evaluation of the correlation between SMS and pollen data.

The results in the CDSS will be developed automatically on the basis of the tuning of the cut-offs related to symptoms and pollens (intensity, duration, pollen peak, whole season, etc.) selected by the user.

Figure 9 has the final representation of the decision pyramid, with coloration of the fourth sector of the faces related to the relevant allergens, in this case graminaceae and parietal and coloring of the central zone, within which are reported the names of the inhalants of interest.

At the end of the 4 steps illustrated before the CDSS system, with the methodology and algorithm of "pyramidal exclusion", allows to obtain the final answer about which are the clinically relevant inhalants for that patient and which can be taken into account for a possible allergen-specific immunotherapy (AIT).

As already anticipated with regard to the symptomatological scores, the instrument will give the doctor the possibility to modulate ("tuning") the minimum cut-offs of positivity of the allergological tests used (e.g., Skin Prick Test 3 - 5 mm; specific IgE dosage from Class 1 to Class 2, severity of symptoms, concentration of pollens, etc.), so that we can reach a more specific diagnosis for a given allergengenic source.

The system, which is the object of this patent application, at the end of the illustrated path will provide for a synthetic report useful to the patient and his doctor for the best therapeutic choices. The report for Case 1, which is the synthesis of the various passages that have been examined analytically previously, includes:

### * Presentation Table of the clinical case

| Case 1, M, 13 aa | | |
|---|---|---|
| Allergic rhinitis | Terapia | Others |
| Persistent | Anti-H1 topic | Allergic familiarity |
| Moderate-Severe | Anti-H1 oral | Atopy for perennial allergens |
| Blocker | Topic steroid | No animals at home |
| Onset age: 7 years | Oral Steroid | |
| Conjunctivitis: no | | |

### Clinical history and pollen calendar concerning Rome

| | | | | | | | | | | | | |
|---|---|---|---|---|---|---|---|---|---|---|---|---|
| ROME | G | F | M | A | M | G | L | A | S | O | N | D |
| CYPRESS | L | H | H | L | L | | | | | L | L | L |
| PARIETARIA | L | L | M | H | H | M | M | L | L | L | L | L |
| HAZEL | L | L | M | M | L | | | | | | | L |
| FAGACEAE | | | | H | H | M | L | | | | | |
| GRAMINEAE | | | L | H | H | H | M | L | L | L | L | |
| OLIVE | | | | | H | H | | | | | | |
| ALTERNARIA | | | | | L | M | M | M | H | L | | |
| AMBROSIA | | | | | | | | | L | | | |
| PATIENT | | | | | | | | | | | | |

| SPT | ALLERGEN | slgE |
|---|---|---|
| 6,0 | PARIETARIA | 18,0 |
| | Par J 2 | 35,0 |
| 10,5 | GRAM (PHL) | 90,0 |
| | Phl p 1 | 80,0 |
| 8,5 | GRAM (CYN) | 2,4 |
| | Cyn d 1 | 0,4 |
| 4,5 | AMBROSIA | 0,1 |
| | Amb a 1 | 0,1 |
| 0,0 | ALTERNARIA | 0,1 |
| | Alt a 1 | 0,0 |
| 3,0 | HAZEL | |
| | Cor a 1 | 0,1 |
| 4,0 | FAGACEAE | 0,1 |
| | BeE v 1 | 0,1 |
| 4,0 | OLIVO | 0,4 |
| | Ole 1 | 0,1 |
| 3,0 | CYPRESS | 0,1 |
| | Cup a 1 | 0,1 |
| PROFILINE | Phl p 12 | 17,4 |
| | Bet v 2 | 29,0 |
| POLCALCINE | Phl p 7 | 0,1 |
| | Bet v 4 | 0,1 |
| CARBOHYDRATES | CCD | 1,0 |

| | |
|---|---|
| 0,0 | ACARUS |
| 6,0 | CAT |
| 3,5 | DOG |

| | |
|---|---|
| * SPT Results and serum IgE assay * Symptoms and medication curves (SMS-Symptoms medication Score) recorded by patients in the monitoring application (see fig. 8) * Final chart in the shape of a pyramid, with coloring of faces relative to the allergens of interest, and of the apical zone, where these are reported (see Fig. 9) | |

From the report we can deduce the presence of suggestive symptoms of allergic rhinitis in MARCH-SETPTEMBER of moderate/severe grade and persistent periodicity. There is a sensitization to perennial allergens.

SPTs are positive for: PAR, GRA, CIP, NOC, AG, and OLE.

Molecular tests confirm the positivity to SPT for GRA, PAR.

There are IgE to PROFILINE and CCD (i.e cross-reactive carboidrates).

The clinical diary supports the diagnosis of allergic rhinitis to GRA, PAR.

### Tuning the system

As mentioned above, the user can choose different thresholds (cut-offs) for the various parameters in order to vary the sensitivity and specificity of the system of his choice.

The following are, for example, some of these values indicated or with the letter "L", which indicates the term "loose", i.e. a less restrictive criterion, with gain in sensitivity, but loss in specificity, or with the letter "S", that is "strong" and therefore a more restrictive criterion that improves the specificity at the expense of sensitivity.

The software, and the underlying algorithm, perform all the various calculations
CALENDAR Pollinic
L: orange and red days
S: only red
SPT (diameter of the allergen pomfo)
L: >= 3 mm
S: >= 5 mm
MOLECULES (IgE sensibilations classes of the molecular test, with the ESEP method)
L: ESEP >= class 1
S: ESEP >= class 2
@M (clinic score used and provided by the electronic diary)
L: average CSMS >1,19
S: average >1,28
-----------
ADH (recording adherence on @M)
L: >=70%
S: >=80%
by expressing the final result in the report's Pyramid system.

Further peculiar characteristics of the preferred forms of realization of the present invention, and which represent a second methodology of analysis, will be described below. In fact, while leaving the same data used in the "Pyramidal exclusion" method, namely: Clinical History, SPT (Regiostrati with Web Allergycard), SMS recorded with Allergymonitor, Total IgE and IgE specific to extracts molecules, pollen count, these data will be processed according to specific algorithms to lead to better diagnostic appropriateness, that is to say the best cause-effect correlation between inhalant allergens (in particular pollen and alternaria) and clinical symptomatology of the patient.

Statistical analyses with the various algorithms can use the numerical data of the curves for a given pollen (e.g. Graminaceae) as they are or multiplied (i.e., corrected) for the value of the level of allergic sensitization of that patient for that Pollen. This level is expressed precisely by the specific activity (SA) of the given patient, which is the ratio between the quantitative value of specific IgE or for the extracts in toto (in this example IgE specific for Phleum pratensis) or of the specific IgE for the molecular allergens of that pollen (e.g. IgE for Phl p1, IgE per phl P5, etc., which can be used as a single or cumulative data, variously combined). Therefore, we will be able to distinguish an SA for extracts and a SA for molecules.

By this way the higher the SA for that pollen, the more the "weight" of the pollen concentration will be greater for that patient. To exemplify: at the same concentration of two pollens (e.g. Phleum and Olea) in the same period, their clinical significance will be different on the basis of the SA of that patient. Imagining for example that the patient is sensitized only to the Phleum but not to the olive, the derived value of the concentration of the olive pollen multiplied by SA relative to the olive = 0, will cancel the values of pollen count of the olive. Therefore, for that patient, even with the same concentration of pollen grains, the only significant pollen will be that of graminacae.

In this way the values of the pollen curves (correct or not through the SA) can not only be visualized individually and compared graphically with the symptoms (expressed with different SMS) but they can be analyzed altogether by means of statistical approaches multivariate. The response of a symptom to the different pollens is not a approachable phenomenon using a deterministic approaches because this response is determined by physiological, biochemical and environmental components (just to make some examples) but with approaches that consider the extreme variability of the signal and therefore with approaches related to probability (probabilistic or stochastic approaches). These approaches take into account the variability of the signal (given by the aforementioned causes) and obtain results more important than the deterministic ones. This approach is one of the novelties of this invention. The multivariate approach also leads to the observation of several variables together (pollens) and displays/models in relation to the variable response (SMS). Usable approaches can be non-supervised (such as the Principal Component Analysis-PCA), where a dependent or supervised variable is not highlighted, where a model seeks the best solution to estimate an employee variable (categorical (classification) or continuous (regression)]. In the first case (non-supervised approaches) we rely on a graphical synthesis where the proximity of the symptom with one or more pollens (corrected with SA or not) indicates the correspondence (analysis of the correspondences). The data can be further synthesized by establishing thresholds where the data itself is considered as present (1) or absent (0) generating a binary matrix (the choice of the threshold can be made on an empirical, statistical, or percentage basis). With these approaches it is also possible to use other types of datasets (weather-climatic, environmental, polluting, etc.) adopting approaches similar to multidimentional scaling (Nnometric-Multi-Dimentional Scaling, 2-block Partial Least Squares, CANOnical COrrespondence, etc.) and display together the different blocks of variables in the same space by highlighting their bindings and their corresponding. The supervised multivariate approaches generate, instead, the application models for the estimation of the symptom expressed in quantitative (regression) or categorical way compared to a threshold (classification). These approaches are very powerful and require strategies to avoid overfittng and establish real forward-looking efficacy (high number of observations and repetitions over several years). In the case of multivariate regression, we can list some approaches: multivariate Linear regression, Principal Component regression, Partial Least Squares regression, Artificial Neural Networks. As far as the multivariate classification is concerned, some approaches can be listed: Partial Least Squares Discriminant Analysis, Soft Independent Modelling of Class Analyogies, Support Vector Machine, Artificial Neural Networks.

The system therefore relies largely on the acquisitions of data described aimed at the study of allergens, and in particular as regards:
1. Allergy history of the patient
2. Results of skin tests
3. Laboratory test results (specific-IgE for extracts and for allergen molecules)
4. Pollinic curves, of the period and of the whole year
5. Meteoclimatic data (provided by ARPA)
6. Environmental pollution data (provided by ARPA)
7. Data recorded by the patient with the application according to the present invention
8. Symptoms Medication Score (SM) derived from the data recorded on the application according to this invention.

The difference with the 1st method of pyramidal exclusion, described above, consists in the elaboration of the aforementioned data with different algorithms aimed at the identification of the most relevant allergen/s for the patient, expressed both in concordance numerical term, both as images with the graphical representation of greater or lesser proximity between the selected SMS, and the graphic representation of the analyzed allergens (e.g. pollens, fungal spores, etc.), or other methodology of more intuitive representation. The present system is also integrated into an application where all the above mentioned data are conveyed and then the result of the relative analysis are presented to the patient and/or physician by algorithms resident on the control unit servers.

But this method does not refer exclusively to pollinic allergens, but also extends to food allergy, medication, insect bites, etc.

The following is a description of these procedures.

Different clinical scores can be used to describe the different types of symptoms, evaluating their presence and/or severity. For example, the following clinical scores, internationally validated:
- RTSS: Rhinoconjunctivitis Total Symptom Score
- ACS: Average Combined Score
- AdSS: Average Adjusted Symptom Score
- RMS: Rescue Medication Score
- dCSMS: Daily Combined Symptoms Medication Score

The following approaches relate to the relationship between symptoms (identified by different indices) and variables linked, for example, to environmental variables (weather-climatic, organic and inorganic particulate present in the air) and/or variables depending on pollens or inhalant allergens. These variables can be corrected and modulated with the physiological state of the patient measured for example with the results of skin prick tests or specific IgE for extracts or allergen molecules or dosage of IgE and/or nasal IgG-specific or other biological fluids, or as a result of nasal and/or conjunctival provocation tests specific for a specific allergen.

Moreover, indices derived from the reciprocal relation between some of these parameters can be taken into account.

In particular, as shown in the practical example described below, the correction of the concentration of a specific pollen for patient-specific sensitization expressed as a Specific Activity (SA) was used as variable.

The SA is given by the ratio of the concentration of the specific IgE measured in that patient to both the allergenic extracts and the allergenic molecules of that pollen (e.g. Phi p1; Phl p5; for Graminaceae), and the concentration of the total IgE of the patient. Obviously higher will be the SA for that allergen the greater will be the sensitization and responsiveness of the patient.

Therefore, the correction of the pollen concentration to which the patient is sensitized has a different weight depending on the SA.

These correct pollen counts were expressed in the example shown as: pc SA-IgE-M, pc SA-IgE-E, respectively for the SA towards the molecules or towards the extracts.

The relationship between symptoms and variables allows the practitioner (physician) to facilitate the prescription of vaccines, indicating, based on the outcome of the multivariate approaches, a better relationship between the symptoms and, for example, the inhalant allergens present in the area in a period of greater symptomatology.

The approaches that can be used may involve sorting techniques (Principal Component Analysis-PCA, Canonical Correspondence Analysis-CCA, Principal Coordinates Analysis-PCooA, etc.) and clustering, both unsupervised, or more complex modelling approaches linked to supervised classification or regression techniques by linear approaches (Linear Discriminant Analysis-LDA, Quadratic Discriminant Analysis-QDA, Partial Least Squares analysis-PLS, etc.) or nonlinear ( Artificial Neural Networks-ANN, Support Vector Machines-SVM, etc.). The matrix of variables and symptoms can be transformed in different ways: standardization, smoothing, binarization by a certain threshold, etc.

The analysis of a real case in which in the analysis were included, only for demonstrative purposes, the pollen curves, the patient's SMS and the sensitization expressed as specific IgE, is reported below, only as an example.

In more details, they were taken into account:
- 3 scores of symptoms and/or symptoms + medications (RTSS, ACS, Adss);
- 4 pollen types (GRA = Graminaceae, Olea = olive, CUP = cupressaceous, URT = Urticaceae);
- 1 spore (ALT = Alternaria);
- 3 types of pollen data: (i) absolute pollen counts; pc AS-IgE-M and pc AS-IgE-E: that is, count pollen corrected for SA both for the allergenic molecules and for the extracts in toto.

The data for pollens/spores (in the 5 different modes) have been corrected in binary (presence/absence) according to a certain percentage (X) of values:
a. Above the X percentage during the whole year
b. Above the X percentage compared to the recording period of symptoms.

This X-value can be chosen by the operator with a dedicated tuning as intended for the method described above.

A concrete example, shown below, shows the application of a univariate concordance approach and a multivariate approach (Analysis of the simple correspondence-CCA) applied to an array of concordance between symptoms and binarized pollens above a preset threshold.

But as previously mentioned, many other approaches can be applied, and even at the same time more than one evaluating which determines the best result.

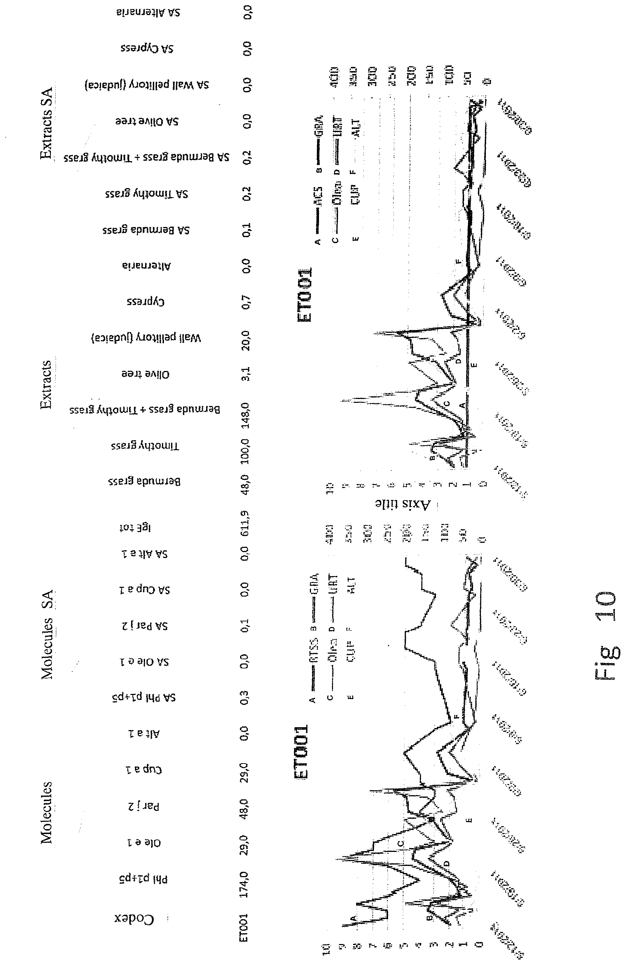

The graph of Fig. 10 shows for a single anonymous patient (ET001), negative only for Alternaria, a symptom score (RTSS) and 5 pollen classes (GRA = Graminaceae, Olea = olive, CUP = Cupressaceous, URT = Urticaceae, ALT = Alternaria) represented in the trend during the registration period by means of the application according to the present invention.

Furthermore we have reported data on specific IgE for molecules and extracts and the derivate SA, which is used by the algorithm for the correction of pollen concentrations as described above.

It can be observed that, from the simple visual analysis of the graph of the RTSS, the symptom follows mainly the evolution of the pollens of Graminaceae, Olea and Urticaceae The following graphs represent the numerical result of the univariate approach (with the numerical value of the concordance index for each analyzed setting), and the graphical result of the multivariate one of the concordance, expressed in terms of proximity, between two analyzed scores, RTSS and ACS, and pollinic variables, taken as absolute or correct for SA referred to the molecular IgE or for the extracts.

In all these graphs, there is a greater proximity to the RTSS point and/or ACS with the three pollen species G (Graminaceae), O (Olea), and U (Urticaceae), or with the correct pollen curves for the SA.

Here is the legend used in the next graphs.

The following is the legend used in subsequent charts.
Legend
G or GRA = Graminaceae pollens
O or OLEA = Olive pollens
U or URT = Parietaria (Urticacea) pollens
C CUP = Cypress pollens
A or ALT = Alternaria spores
As = specific activity = IgE concentration (for extracts, or for molecules) / total IgE pc SA = SA correct pollinic curves
mG = pc SA-IgE M Graminacae = concentration of pollen curves correct for the SA of Molecular IgE of the Graminacae
mO = pc SA-IgE M Olive = concentration of pollen curves correct for the SA of Molecular IgE of the Olive
mU = pc SA-IgE M Parietaria= concentration of pollen curves correct for the AS of Molecular IgE of the Parietaria
mC = pc AS-IgE M Cypress = concentration of pollen curves correct for the SA of Molecular IgE of the Cypress
mA = pc SA-IgE M Alternaria = concentration of pollen curves correct for the SA of Molecular IgE of the Alternaria
eG = pc SA-IgE M Gramineae Extracts = concentration of pollen curves correct for the AS of Molecular IgE of the Gramineae Extracts
eO = pc SA-IgE M Olive Extracts = concentration of pollen curves correct for the SA of Molecular IgE of the Olive Extracts
eU = pc SA-IgE M Paretaria Extracts = concentration of pollen curves correct for the SA of Molecular IgE of the the extract of Parietaria
eC = pc SA-IgE M Cypress Extract = concentration of pollen curves correct for the SA of Molecular IgE of the the extract of Cypress
eA = pc SA-IgE M Alternaria Extracts = concentration of pollen curves correct for the SA of Molecular IgE of the extract of Alternaria

Multivariate analyses provide for an interpretation that takes into account not only the relationship between symptom and single pollinic species but considers the relation (multivariate) among all the pollinic species together. This analysis, although it may seem complex, actually is more "natural" since even the abundance of pollen species is not disconnected. Multivariate charts provide a multivariate profile which is nothing more than a two-dimensional space disposition (most commonly) or tri-dimensional disposition of the variable symptom (theoretically dependent) together with other variables (pollens in the specific case, but also environmental). This multivariate profile should be read as a geometric proximity variable symptom referred to other variables by considering first multivariate axis as the most important (due to the nature of the algorithm used).

The two charts below show two examples of multivariate processing of anonymous patients (pt ET001 and pt ET059).

For each patient, the line diagram commonly used by doctors to carry out assessments of the sensitivity of the patients based on the symptoms that are registered with Allergymonitor (the electronic diary) is represented at the top. This diagram shows together the pollen counts and symptom of various species (GRA = Gramineae, Olea = Olive, CUP = Cupressacee, URT = Urticaceae, ALT = Alternaria). Dashed circles on the graph (manually arranged) show the spikes in symptoms characterized by a greater criticality. At the bottom on the right it is showed the result of a multivariate analysis of the correspondences represented with a scatter plot of the first axis of the analysis. In this analysis we note the proximity of the symptom referred to the various pollinic species. In the patient ET001 is highlighted a polisensivity for four pollen types considered (GRA, URT, Olea, CUP) while there is no sensitization to Alternaria (ALT) spore. Even in the patient ET059 a polisensivity is observed for four pollen types considered (GRA, URT, Olea, CUP) as well as for the Alternaria (ALT) spore; patient ET059 sensibility for Olive and Cupressacee is less than other molecules considered.

Also the following diagrams 15 and 16 show pie charts, in which we observe the positivity of the patient considered allergens tested, by checking the values of the specific present IgE.

Table 1 is an index showing the correlation between symptom and single pollen species.

**Tabella 1 Concordance index**

| GRA | Olea | URT | CUP | ALT |
|---|---|---|---|---|
| 33 | 33 | 33 | 35 | 21 |

The reading of this table shows that the highest concordance values represent a higher association between symptom and pollen. Although this analysis is univariate, it allows a comparison (and an additional information) compared to multivariate analysis. This approach can help to provide a kind of significance, which otherwise, in the multivariate field, would be impossible to have.

From an overall observation of the numerical output and the graphic output for the two patients there is a convergence in the interpretation of the symptoms and how they are related to the individual pollinic species.

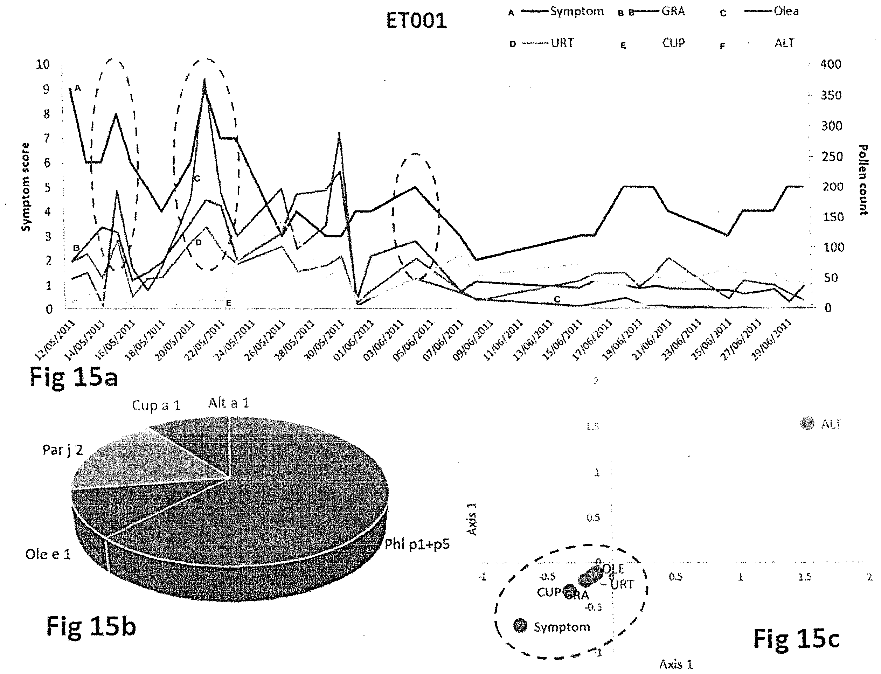

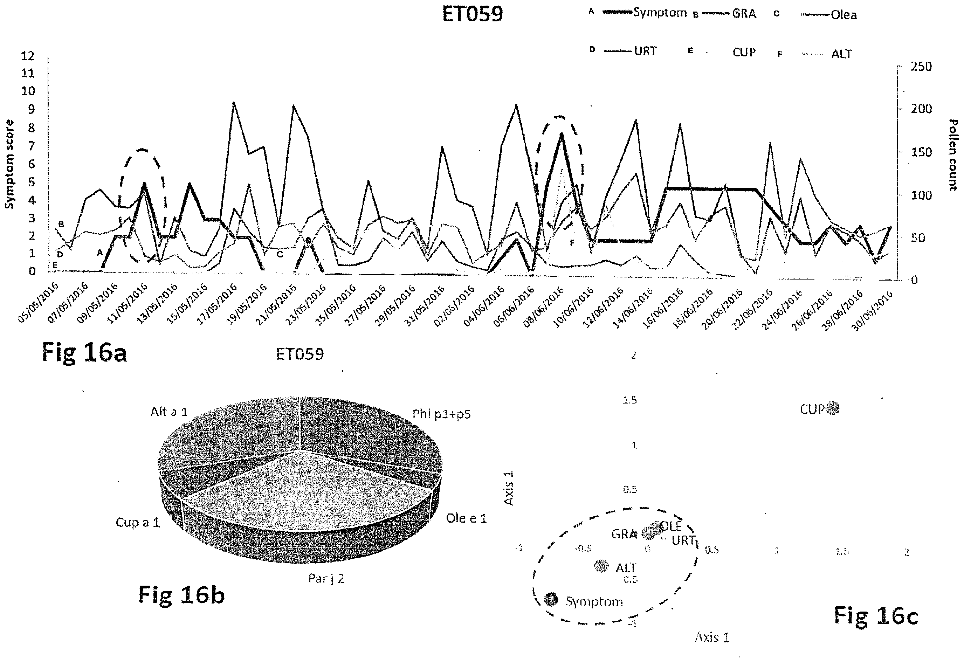

In particular, the molecular values and the values of extracts for the patient ET001 show a polysensibilization for the four types of pollen considered (graminaceae, urticaceae, olive, cupressaceous) while there is no sensitization to Alternataria spores (both values = 0.00), as shown in the 15b pie chart, which shows the contributions of the different molecules of the specific IgE to which the patient is sensitive. The diagram in Fig. 15a with a line chart, three dashed circles on the chart (manually traced) show the peaks of symptoms with greater criticality and correspond to the pollen peaks of the taxa to which the patient is sensitized and more present in the considered period: graminaceae, urticaceae, olive. In the figure at the bottom on the right, there is the 15c result of the multivariate analysis of the correspondences represented with a scatter plot of the first axis of the analysis: the proximity of the symptom is noted compared to the three pollinic species-graminaceae, urticaceae, olive- for which the patient is more sensitized, according to the previous graph. Table 2 - with reference to the representations shown in Figures 16a, 16b, 16c, shows the numerical values of the correlation index between the symptom and the pollinic species.

**Table 2 Concordance Index**

| GRA | Olea | URT | CUP | ALT |
|---|---|---|---|---|
| 27 | 23 | 37 | 27 | 29 |

The molecular values and extracts for the patient ET059 show a polysensibilization for the four types of pollen considered (graminaceae, urticaceae, olive, cupressaceous) as well as for the alternaria spore; sensitization of the patient ET059 for olives and cupressaceae is lower than the other molecules considered (as shown in the 16b pie chart). In line chart 16a the two dashed circles on the chart (manually arranged) show the peaks of symptoms with greater criticality and correspond to the pollen peaks of the taxa to which the patient is sensitized and more present in the period considered: graminaceae and urticaceae, in the first period, urticaceae and alternaria graminaceae, in the second period. In the figure at the bottom on the right there is the result of the multivariate analysis of the correspondences represented with a scatter plot of the first axis of the analysis: it is pointed out the proximity of the symptom referred to pollens and the alternaria spore, in the sequence: alternaria, graminaceae, urticaee, olive and finally cupressaceous; the latter at a greater distance, since, the relative pollens have relatively low concentrations, during the period considered, contributing in a reduced way to the symptomatology of the patient. The numerical values of the correlation index between the symptom and the pollinic species can be observed in the table.

### Reference

1. Dondi A., Tripodi S., et Al. Pollen-induced allergic rhinitis in 1360 Italian children: Comorbidities and determinants of severity. Pediatr Allergy Immunol 2013: 24: 742-751
2. Stringari G., Tripodi S. et al, The effect of component-resolved diagnosis on specific immunotherapy prescription in children with hay fever , J Allergy Clin Immunol. 2014 Jul;134(1):75-81
3. Bianchi A., Tsilochristou O., Gabrielli F., Tripodi S., Matricardi PM. The Smartphone: A Novel Diagnostic Tool in Pollen Allergy? Investig Allergol Clin Immunol. 2016;26(3):204-7.
4. O.Pfaar et al. Defining pollen exposure times for clinical trials of allergen immunotherapy for pollen-induced rhinoconjunctivitis. Allergy 72 (5), 713-722. 2017 Jan 27

## Claims

1. A multivariate method for providing a clinical diagnostic support for allergic diseases, comprising the steps of
a) acquiring data relating to:
i. an allergy history of a patient by recording the seasonality of symptoms,
ii. skin allergy test results,
iii. laboratory test results of specific IgEs for allergen extracts and/or for allergenic molecules,
iv. a local pollinic calendar,
v. meteorological data,
vi. environmental pollution data;
b) assigning a Symptoms Medication Score (SMS) derived from the data acquired in steps i., ii. and iii. in the form of a Rhinoconjunctivitis Total Symptom Score (RTSS), Average Combined Score (ACS), Averge Adjusted Symptom Score (AdSS), Rescue Medication Score (RMS) or Daily Combined Symptoms Medication Score (dCSMS),
c) detecting the most relevant allergen (s) for the patient by means of a computer based decisional procedure based on the assigned Symptoms Medication Score (SMS), correlated with the data acquired in steps iv., v. and vi., wherein the computer based decisional procedure includes Principal Component Analysis (PCA), Canonical Correspondence Analysis (CCA), Principal Coordinates Analysis (PCooA) and/or clustering and/or Linear Discriminant Analysis (LDA), Quadratic Discriminant Analysis (QDA), Partial Least Square Analysis (PLS) and/or Artificial Neural Networks (ANN) and/or Support Vector Machines (SVM),
d) wherein detecting the most relevant allergen for the patient comprises correcting data for a pollen/inhalant allergen concentration by the Specific Activity (SA) of pollen/inhalant allergen for the patient, wherein said Specific Activity being the ratio of specific IgEs concentration detected in the patient with both allergenic extracts and allergenic molecules of selected pollen/inhalant allergens, to the total IgE concentration for that patient.

2. The method according to claim 1, **characterized in that** a corrected pollen/inhalant allergens counts is expressed as pollens/inhalant allergens counts corrected by the Specific Activity (SA) respectively related to molecules (SA-IgE-M pc) or extracts (SA-lgE-E pc).

3. The method according to any of the preceding claims, **characterized in that** a variables and symptoms' matrix is created which is optionally transformed via standardization and/or smoothing and/or binarization techniques.

4. The method according to any of the preceding claims, **characterized in that** environmental data and/or pollens/inhalant data are corrected and modulated with respect to patient sensitization physiological state, is the patient sensitization physiological state being measured by allergological tests, the correction and modulation being carried out by multiplying both the IgE specific activity with the pollen/inhalant allergen concentration -> IgE-pc (= IgE-SA * pollen concentration), and the extract specific activity (Extract-SA) with the pollen concentration (= Extract-SA * pollen concentration).

5. The method according to any of the preceding claims, **characterized in that** data relating to environmental variables including weather and climate-related variables and organic and inorganic particulates present in the air are acquired and processed as well.

6. The method according to any of the preceding claims, **characterized in that**:
i. different Symptoms Medication Scores (SMS), which are recorded either as symptom evolution and/or drug effects in patients, are correlated with the curves of inhalant allergens, pollen and molds, based on patient clinical history and sensitizations; and
ii. previous examinations results are integrated and represented through a geometric figure of a three-dimensional ideal solid with a regular base and configured as a polyhedral angle, consisting in a pyramid having a base with n sides, wherein n is equal to the number of the investigated allergens, and faces with different sectors, each sector being dedicated to one of the four diagnostic steps (i- clinical history and allergen inhalant curves ii- SPT results, iii- Component Resolved Diagnosis (CRD), iv- SMS), arranged in order from said pyramid base to apex, wherein to each allergen is assigned a color, the intensity of which increases in a centripetal direction, according to the increased sensitivity of diagnostic tests, at the end of the diagnostic process, providing by a CDSS system, for a synthetic report, useful to both a patient and a treating physician for the best therapeutic choices, such report including a clinical case description, results of the different diagnostic phases, a final pyramid-shaped chart, in which only faces related to interested allergens will be colored -not gray- and said allergens will be indicated at the center of said chart, and a brief descriptive summary of results obtained at the different phases.

7. The method according to any of the preceding claims, **characterized by** having both sensitivity and specificity of a final response adjustable by a user through a tuning procedure which allows to vary the cut-offs for different tests according to a restrictive criterion, named "strong", or a less restrictive criterion, named "loose".

## Patentansprüche

1. Multivariates Verfahren zur Bereitstellung einer klinischen Diagnoseunterstützung für allergische Erkrankungen, das die folgenden Schritte umfasst
a) die Beschaffung von Daten in Bezug auf:
i. eine Allergieanamnese eines Patienten durch Aufzeichnung der Saisonalität der Symptome,
ii. Ergebnisse von Hautallergietests,
iii. Labortestergebnisse spezifischer IgEs für Allergenextrakte und/oder für allergene Moleküle,
iv. einen lokalen Pollinenkalender,
v. meteorologische Daten,
vi. Daten zur Umweltverschmutzung;
b) Zuweisung eines Symptom-Medikations-Scores (SMS), der aus den in den Schritten i., ii. und iii. erfassten Daten abgeleitet wird, in Form eines Rhinokonjunktivitis-Gesamtsymptom-Scores (RTSS), eines durchschnittlichen kombinierten Scores (ACS), eines mittelwertbereinigten Symptom-Scores (AdSS), eines Rescue-Medikations-Scores (RMS) oder eines täglichen kombinierten Symptom-Medikations-Scores (dCSMS),
c) Erkennen des/der relevantesten Allergene(s) für den Patienten mittels eines computergestützten Entscheidungsverfahrens auf der Grundlage des zugewiesenen Symptom-Medikations-Scores (SMS), korreliert mit den in den Schritten iv., v. und vi. erfassten Daten, wobei das computergestützte Entscheidungsverfahren die Hauptkomponentenanalyse (PCA), die kanonische Korrespondenzanalyse (CCA), die Hauptkoordinatenanalyse (PCooA) und/oder das Clustering und/oder die lineare Diskriminanzanalyse (LDA), die quadratische Diskriminanzanalyse (QDA), die Partial-Least-Square-Analyse (PLS) und/oder künstliche neuronale Netze (ANN) und/oder Support Vector Machines (SVM) umfasst,
d) wobei der Nachweis des relevantesten Allergens für den Patienten das Korrigieren der Daten für eine Pollen-/ Inhalationsmittelallergenkonzentration um die spezifische Aktivität (SA) des Pollen-/Inhalationsmittelallergens für den Patienten umfasst, wobei die spezifische Aktivität das Verhältnis der spezifischen IgE-Konzentration, die bei dem Patienten sowohl mit allergenen Extrakten als auch mit allergenen Molekülen ausgewählter Pollen-/Inhalationsmittelallergene nachgewiesen wurde, zu der gesamten IgE-Konzentration für diesen Patienten ist.

2. Verfahren nach Anspruch 1, **dadurch gekennzeichnet, dass** eine korrigierte Pollen-/Inhalationsallergenzahl als Pollen-/Inhalationsallergenzahl ausgedrückt wird, die um die spezifische Aktivität (SA) korrigiert wird, die jeweils auf Moleküle (SA-IgE-M pc) oder Extrakte (SA-lgE-E pc) bezogen ist.

3. Verfahren nach einem der vorhergehenden Ansprüche, **dadurch gekennzeichnet, dass** eine Variablen- und Symptommatrix erstellt wird, die optional durch Standardisierungs- und/oder Glättungs- und/oder Binarisierungstechniken transformiert wird.

4. Verfahren nach einem der vorhergehenden Ansprüche, **dadurch gekennzeichnet, dass** Umweltdaten und/oder Pollen/Inhalationsdaten in Bezug auf den sensibilisierungsphysiologischen Zustand des Patienten korrigiert und moduliert werden, wobei der sensibilisierungsphysiologische Zustand des Patienten durch allergologische Tests gemessen wird, wobei die Korrektur und Modulation durch Multiplikation sowohl der spezifischen IgE-Aktivität mit der Pollen/Inhalationsallergenkonzentration IgE-pc (= IgE-SA * Pollenkonzentration) als auch der spezifischen Aktivität des Extrakts (Extract-SA) mit der Pollenkonzentration (= Extract-SA * Pollenkonzentration) durchgeführt wird.

5. Verfahren nach einem der vorhergehenden Ansprüche, **dadurch gekennzeichnet, dass** auch Daten in Bezug auf Umweltvariablen, einschließlich wetter- und klimabezogener Variablen und in der Luft vorhandener organischer und anorganischer Partikel, erfasst und verarbeitet werden.

6. Verfahren nach einem der vorhergehenden Ansprüche, **dadurch gekennzeichnet, dass**:
i. verschiedene Symptom-Medikamenten-Scores (SMS), die entweder als Symptomentwicklung und/oder Medikamentenwirkung bei Patienten erfasst werden, mit den Kurven von Inhalationsallergenen, Pollen und Schimmelpilzen auf der Grundlage der klinischen Vorgeschichte und Sensibilisierungen der Patienten korreliert werden; und
ii. die Ergebnisse früherer Untersuchungen integriert werden und durch eine geometrische Figur eines dreidimensionalen idealen Körpers mit regelmäßiger Basis und in Form eines polyedrischen Winkels dargestellt werden, der aus einer Pyramide mit einer Basis mit n Seiten besteht, wobei n gleich der Anzahl der untersuchten Allergene ist, und Flächen mit verschiedenen Sektoren, wobei jeder Sektor einem der vier Diagnoseschritte (i- klinische Anamnese und Allergen-Inhalationskurven, ii- SPT-Ergebnisse, iii- komponentenaufgelöste Diagnose (CRD), iv- SMS) gewidmet ist, die in der Reihenfolge von der Pyramidenbasis zur Spitze angeordnet sind, wobei jedem Allergen eine Farbe zugewiesen wird, deren Intensität in zentripetaler Richtung entsprechend der erhöhten Empfindlichkeit der diagnostischen Tests am Ende des diagnostischen Prozesses zunimmt, wodurch durch ein CDSS-System ein synthetischer Bericht bereitgestellt wird, der sowohl für einen Patienten als auch für einen behandelnden Arzt im Hinblick auf die besten therapeutischen Entscheidungen nützlich ist, wobei ein solcher Bericht eine klinische Fallbeschreibung, die Ergebnisse der verschiedenen diagnostischen Phasen, ein abschließendes pyramidenförmiges Diagramm, in dem nur Flächen, die sich auf interessierende Allergene beziehen, farbig - nicht grau - sind und die Allergene in der Mitte des Diagramms angezeigt werden, und eine kurze beschreibende Zusammenfassung der in den verschiedenen Phasen erhaltenen Ergebnisse enthält.

7. Verfahren nach einem der vorhergehenden Ansprüche, **dadurch gekennzeichnet, dass** sowohl die Sensitivität als auch die Spezifität einer endgültigen Antwort durch einen Benutzer mittels eines Abstimmungsverfahrens einstellbar sind, das es ermöglicht, die Grenzwerte für verschiedene Tests nach einem restriktiven Kriterium, "stark" genannt, oder einem weniger restriktiven Kriterium, "locker" genannt, zu variieren.

## Revendications

1. Procédé multivarié visant à fournir un support de diagnostic clinique pour des troubles allergiques, comportant les étapes suivantes :
a) acquérir des données concernant :
i) un historique d'allergie d'un patient, rapportant la saisonnalité des symptômes,
ii) des résultats de tests cutanés d'allergie,
iii) des résultats de tests en laboratoire d'IgE spécifiques d'extraits d'allergène et/ou de molécules allergènes,
iv) un calendrier pollinique local,
v) des données météorologiques,
vi) des donnés de pollution de l'environnement ;
b) attribuer un score symptômes-médication (SMS) dérivé des données acquises dans les étapes (i), (ii) et (iii), sous forme d'un score symptomatique total de rhino-conjonctivite (SSTR), d'un score combiné moyen (SCM), d'un score symptomatique ajusté moyen (SSAM), d'un score de médication de secours (SMSc) ou d'un score symptômes-médication combiné journalier (SMSCJ),
c) détecter le ou les allergène(s) le(s) plus pertinent(s) pour le patient, au moyen d'une procédure informatisée de décision, reposant sur le score symptômes-médication (SMS) attribué, corrélé avec les données acquises dans les étapes (iv), (v) et (vi), étant entendu que la procédure informatisée de décision englobe l'analyse en composantes principales (ACP), l'analyse canonique des correspondances (ACC), l'analyse en coordonnées principales (ACoP) et/ou la classification et/ou l'analyse discriminante linéaire (ADL), l'analyse discriminante quadratique (ADQ), l'analyse par moindres carrés partiels (PLS) et/ou les réseaux de neurones artificiels (RNA) et/ou les séparateurs à vaste marge (SVM),
d) étant entendu que détecter l'allergène le plus pertinent pour le patient comprend le fait de corriger les données de concentration de pollens et/ou d'allergènes inhalés par l'activité spécifique (AS) de pollens et/ou d'allergènes inhalés pour le patient, laquelle activité spécifique est le rapport de la concentration d'IgE spécifiques détectée chez le patient, avec à la fois des extraits d'allergène et des molécules allergènes de pollens et/ou d'allergènes inhalés sélectionnés, à la concentration totale d'IgE chez ce patient.

2. Procédé conforme à la revendication 1, **caractérisé en ce que** l'on exprime un décompte corrigé de pollens et/ou d'allergènes inhalés comme un décompte de pollens et/ou d'allergènes inhalés corrigé au moyen de l'activité spécifique (AS) concernant respectivement les molécules (cp AS-IgE-M) ou les extraits (cp AS-IgE-E).

3. Procédé conforme à l'une des revendications précédentes, **caractérisé en ce que** l'on crée une matrice de variables et de symptômes qui est, en option, transformée à l'aide de techniques de normalisation et/ou de lissage et/ou de binarisation.

4. Procédé conforme à l'une des revendications précédentes, **caractérisé en ce que** l'on corrige et module les données d'environnement et/ou les données de pollens et/ou d'allergènes inhalés en tenant compte de l'état physiologique de sensibilisation du patient, on mesure cet état physiologique de sensibilisation du patient au moyen de tests allergologiques, et l'on effectue correction et modulation en multipliant à la fois l'activité spécifique d'IgE par la concentration de pollens et/ou d'allergènes inhalés, ce qui donne une cp-IgE (soit AS-IgE * concentration de pollens), et l'activité spécifique concernant les extraits (AS-extraits) par la concentration de pollens (soit AS-extraits * concentration de pollens).

5. Procédé conforme à l'une des revendications précédentes, **caractérisé en ce que** l'on acquiert et traite des données concernant aussi bien des variables environnementales, y compris des variables en relation avec le climat et la météorologie, que des particules organiques et inorganiques présentes dans l'air.

6. Procédé conforme à l'une des revendications précédentes, **caractérisé en ce que** :
i) différents scores symptômes-médication (SMS), qui sont enregistrés en tant qu'évolution de symptôme et/ou effets de médicament chez des patients, sont corrélés avec les courbes d'allergènes inhalés, pollens et moisissures, sur la base de l'historique clinique et des sensibilisations de patients,
ii) et les résultats d'examens antérieurs sont intégrés et représentés à travers une figure géométrique d'un solide tridimensionnel idéal présentant une base régulière et configuré avec un angle polyèdre, consistant en une pyramide dotée d'une base à n côtés, n étant égal au nombre d'allergènes objets d'investigation, et de faces comportant différents secteurs, chaque secteur étant dédié à l'une des quatre étapes de diagnostic ( i : historique clinique et courbes d'allergènes inhalés ; ii : résultats de tests SPT ; iii : diagnostic par analyse de composants (CRD) ; iv : SMS), disposés en ordre de la base à la pointe de ladite pyramide, étant entendu qu'à chaque allergène est attribuée une couleur dont l'intensité augmente dans la direction centripète, selon la sensibilité accrue des tests de diagnostic, à la fin du processus de diagnostic, fournissant par un système CDSS un rapport de synthèse utile à la fois à un patient et à un médecin traitant en vue des meilleurs choix thérapeutiques, un tel rapport incluant une description de cas clinique, les résultats des différentes phases de diagnostic, un diagramme final en forme de pyramide dans lequel seules les faces en relation avec les allergènes intéressants seront colorées - et non pas grises - et lesdits allergènes seront indiqués au centre dudit diagramme, et un bref résumé descriptif des résultats obtenus dans les différentes phases.

7. Procédé conforme à l'une des revendications précédentes, **caractérisé en ce qu'**il présente à la fois une sensibilité et une spécificité de la réponse finale qui peuvent être ajustées par l'utilisateur grâce à une procédure de réglage qui permet de faire varier les seuils de coupure pour différents tests selon un critère restrictif, dit « fort », ou un critère moins restrictif, dit « faible ».
